# EUROPÄISCHE PATENTSCHRIFT

(11) **EP 2 482 865 B1**
(45) Veröffentlichungstag und Bekanntmachung des Hinweises auf die Patenterteilung: **06.05.2015**
(21) Anmeldenummer: 10765952.6
(22) Anmeldetag: 30.09.2010
(51) Int. Cl.: A61M 1/10

(54) **Herzunterstützungsvorrichtung**
Cardiac assistance device
Dispositif d'assistance cardiaque

(30) Priorität: 30.09.2009 DE 102009043795
(43) Veröffentlichungstag der Anmeldung: 08.08.2012
(73) Patentinhaber: AdjuCor GmbH, 85748 Garching (DE)
(72) Erfinder: MEISTER, Markus, 80997 München (DE); WILDHIRT, Stephen, 80997 München (DE)
(74) Vertreter: Peterreins, Frank
(86) Internationale Anmeldenummer: PCT/EP2010/005947
(87) Internationale Veröffentlichungsnummer: WO 2011/038904

(56) Entgegenhaltungen:
- EP-B1- 1 748 809
- WO-A1-00/25842
- WO-A1-98/30271
- US-A- 5 749 839
- US-A1- 2002 065 449
- US-A1- 2008 214 888
- US-B1- 6 602 182
- US-B1- 6 626 821

## Beschreibung

Die Erfindung betrifft eine Herzunterstützungsvorrichtung und ein Verfahren zu ihrer Steuerung.

Das Krankheitsbild der Herzinsuffizienz (engl.: Heart Failure) beschreibt den Zustand der Unfähigkeit des Herzens, durch ausreichende Pumpkraft den Organismus mit Blut zu versorgen. Es zeichnet sich durch ein klinisches Syndrom bestehend aus Müdigkeit, Kurzatmigkeit und Flüssigkeitsretention aus. Die Ursachen sind vielschichtig und korrelieren oft mit den Lebensgewohnheiten (Risikofaktoren) der Industrienationen. Epidemiologisch lassen die USamerikanischen Zahlen das Ausmaß der Erkrankung erkennen und sind in vollem Umfang auf die sieben wichtigsten Medizin und Gesundheitsmärkte (USA, Frankreich, Deutschland, Italien, Japan, Spanien und England) anwendbar. Generell wird davon ausgegangen, dass weltweit 23 Mio. Menschen an Herzinsuffizienz erkrankt sind und ca. 2 Mio. Neuerkrankungen pro Jahr hinzu kommen.

In den USA leiden derzeit 5 Mio. Menschen an Herzinsuffizienz, mit einer jährlichen Neuerkrankungsrate von 550.000. Aufgrund der sich dramatisch entwickelnden Altersstruktur wird sich in den nächsten zehn Jahren die Anzahl der herzinsuffizienten Patienten in jeder Lebensdekade jenseits des 50. Lebensjahres verdoppeln; das heißt, in den USA werden im Jahre 2018 insgesamt 10 Mio. Menschen an dieser Erkrankung leiden und eine entsprechende Therapie in Anspruch nehmen müssen.

Um die Lebenserwartung des Patienten zu erhöhen, gibt es neben der Herztransplantation oder der Implantation intravaskuläre Unterstützungssysteme mit den damit verbundenen Risiken des Schlaganfalles, Blutungen oder Infektionen auch Überlegungen, um das Herz eine Manschette oder einen Sack zu legen, der aufblasbare Kammern aufweist. Diese Kammern werden entsprechend der Herzkontraktion befüllt und entleert, sodass sie von außen gegen das Herz drücken und es beim während der Kontraktion durch aktives Komprimieren oder augmentieren vollständig oder zumindest partiell unterstützen oder sogar die Herztätigkeit übernehmen. Der Sack oder die Manschette sind möglichst flexibel, um sich an die Form des Herzens anpassen zu können. Ein Beispiel für eine solche Herzunterstützungsvorrichtung gibt die EP 1 748 809 B1.

US 6,626,821 B1 offenbart eine Vorrichtung mit über eine Pumpe aufblasbaren Elementen. Die Pumpe beinhaltet ein Reservoir zum Speichern von rückkehrendem Fluid zwischen den Befüllungszyklen.

US 2008/0214888 A1 offenbart eine Herzunterstützungsvorrichtung mit einem geschlossenen Fluidsystem. Die Vorrichtung umfasst aufblasbare Einheiten und eine Fluidpumpe.

US 2002/0065449 D8 offenbart eine impläntierbare Vorrichtung mit Hilfe derer die Ausdehnung des Ventrikels durch Aufbauen von Druck in aufblasbaren Kissen beschränkt werden kann.

Die Erfindung betrifft eine leistungsfähige Herzunterstützungsvorrichtung gemäß den Ansprüchen. Die erfindungsgemäße Herzunterstützungsvorrichtung umfasst einen intrakorporalen Fluidnachfülltank (84) und ein intrakorporales Nachfülltankventil (82), das bei Fluidverlust im Fluidkreislauf selbsttätig in die offene Stellung gebracht werden kann.

Die erfindungsgemäße Herzunterstützungsvorrichtung hat ein das Herz umschließendes Trägerbauteil, an dem innenseitig mehrere benachbarte inflatierbare und deflatierbare Kammern vorgesehen sind, über die eine innenseitige Wand einwärts verschiebbar ist, wobei die Kammern über Leitungen mit wenigstens einer Pumpe in Strömungsverbindung stehen. Mehrere zuströmseitige Zuströmventile sind zwischen Pumpe und Kammern angeordnet. Zumindest einzelne Kammern oder Gruppen von Kammern sind eigenen Zuströmventilen zugeordnet, um die Kammern unabhängig einzeln bzw. gruppenweise anzusteuern. Alle Kammern sind vorzugsweise parallel geschaltet, so dass sie einzeln oder in Gruppen ansteuerbar sind. Somit lassen sich auch zeitlich abgestaffelte oder unterschiedliche Druckverläufe realisieren.

Durch die vorzugsweise 1:1 Zuordnung von Kammern und Zuströmventilen lässt sich die Unterstützung des Herzens mittels der erfindungsgemäßen Vorrichtung sehr exakt vornehmen und die natürliche, teilweise sehr komplexe Pumpbewegung des Herzens nachbilden. Ferner können auch gezielt geschädigte Teilabschnitte des Herzens unterstützt werden. Die vorliegende Erfindung sieht vor, nicht nur ein Zuströmventil für die mehreren Kammern bereitzustellen, sondern eine individuelle Ansteuerung der Kammern zu ermöglichen, was mit einer Vielzahl von Zuströmventilen erreicht wird. Damit kann das erkrankte Herz wesentlich individueller unterstützt werden, und es lässt sich ein auf verschiedene Belastungssituationen hin angepasstes Pumpverhalten erzielen, indem die Kammern unterschiedlich stark, auch temporär versetzt zueinander, befüllt werden. Ferner können einzelne Herzregionen segmental (regional) unterstützt werden. Im Stand der Technik sind keine Ventile vorgesehen, weil die Steuerung über Kolbenpumpen als ausreichend erachtet wurde. Die Erfindung setzt sich deshalb deutlich von diesen einfacheren Systemen ab. Die Kammern sind insbesondere asymmetrisch angeordnet.

Zwischen der Pumpe und den Kammern ist zuströmseitig wenigstens ein Druckspeicher angeordnet, in den die Pumpe Druckfluid einspeist, wobei die Ventile stromabwärts des Druckspeichers angeordnet sind. Durch Vorsehen eines Druckspeichers lässt sich die Pumpe bei geringerer Leistung permanent betreiben, und Druckabfälle werden vermieden. Über den Druckspeicher steht permanent genügend Druck zur Verfügung, um sehr schnell einzelne oder alle Kammern zu inflatieren. Zusätzlich oder alternativ hierzu kann auch stromaufwärts der Pumpe eine Druckfluidsammelkammer vorhanden sein, in die die von den einzelnen Kammern kommenden Leitungen münden. Die Druckfluidsammelkammer dient als Fluidzwischenspeicher.

Eine bevorzugte Ausführungsform sieht vor, dass ein den Druck im Druckspeicher ermittelnder Drucksensor vorgesehen ist. Die über den Drucksensor gewonnen Daten erlauben es, die Pumpe gezielt anzusteuern, wenn ein zu hoher oder zu niedriger Druck im Druckspeicher vorhanden sein sollte. Die Pumpe lässt sich somit energiesparend auf minimaler Drehzahl halten.

Bei Unterschreiten/Überschreiten eines vorgegebenen Druckminimums bzw. Druckmaximums im Druckspeicher wird über die Steuerung die Drehzahl der Pumpe verändert.

Wenn von jeder Kammer eine eigene, nur ihr zugeordnete Zu- und Abströmleitung ausgeht, lässt sich auch das Deflatieren individuell für jede Kammer steuern.

Die Kammern sollten unmittelbar mit der Pumpe abströmseitig strömungsverbunden sein, damit ohne wesentliche Strömungsverluste das Fluid sofort wieder über die Pumpe unter Druck gesetzt wird. Damit soll auch die Leitungslänge des Fluidkreislaufes gering gehalten werden.

Es ist von Vorteil, wenn abströmseitig zwischen den Kammern und der Pumpe Abströmventile vorgesehen sind, über die dann die Zeitdauer, über die die Kammern inflatiert bleiben, veränderbar ist. Darüber hinaus lässt sich so auch das Deflatieren der Kammern zeitlich abstaffeln.

Auch die Abströmventile sollten zur Erzielung einer individuelleren Herzunterstützung einzelnen oder Gruppen von Kammern zugeordnet sein.

Eine platzsparende Ausführungsform der Erfindung sieht vor, dass zumindest ein, vorzugsweise alle je einer einzelnen oder einer Gruppe von Kammern zugeordneten Zuström- und Abströmventile zu je einem Mehrwegventil zusammengefasst sind.

Die kammerseitige Zuströmleitung bildet gemäß einer Ausführungsform abschnittsweise die Abströmleitung. Die Kammern selbst haben folglich nur eine gemeinsame Zu- und Abströmleitung, um den Platzbedarf für die Vorrichtung gering zu halten.

Die erfindungsgemäße Vorrichtung lässt sich als geschlossener Kreislauf ausführen, der komplett intrakorporal liegt, oder der teilweise auch extrakorporal positioniert ist (z.B. Pumpe oder Energiespeicher). Eine weitere Ausführungsform der Erfindung sieht vor, die Vorrichtung als offenes System zu betreiben. Dies bedeutet, dass die Vorrichtung nach den Kammern über eine aus dem Körper herausragende Leitung die Druckluft nach außen ablässt. Falls vorhanden, können die Abströmventile noch intrakorporal liegen. Die intra- oder extrakorporal liegende Pumpe saugt Luft aus der Atmosphäre an.

Eine Steuerung der Ventile und eine damit gekoppelte Notfallsensorik erlauben es, bei Detektion einer Leckage einer Kammer die der defekten Kammer zugeordneten Ventile bleibend zu schließen. Da das Nachfüllen von Druckfluid oder, allgemeiner, von Fluid in den Kreislauf sehr aufwendig ist oder gegebenenfalls nur transkutan erfolgt, sollten auch kleinste Leckagen vermieden werden.

Weil der Ausfall einer Pumpe bei einem stark geschwächten Herz lebensbedrohlich sein könnte, sieht die Erfindung gemäß einer bevorzugten Ausführungsform vor, dass eine zusätzlich vorhandene Notfallpumpe aktiviert wird, sobald eine Steuerung der Pumpen die Detektion eines Pumpendefekts erkennt.

Mit einer Steuerung für die Ventile und/oder die Pumpe gekoppelte Durchfluss oder Drucksensoren erlauben es, Druck in einzelnen oder Gruppen von Kammern zu ermitteln. Über diese Werte lässt sich der herzustellende, gewünschte Kammerdruck steuern oder sogar regeln. Ebenso lassen sich Druckabfälle zum Beispiel bei einer Leckage schnell ermitteln. Diese Idee lässt sich, verallgemeinert, sogar auf eine Herzunterstützungsvorrichtung nach dem Oberbegriff des Anspruchs 1 anwenden.

Herzseitige Drucksensoren sollten gemäß der bevorzugten Ausführungsform ebenfalls vorgesehen sein. Sie sind mit einer Steuerung für Ventile und/oder die Pumpe gekoppelt und lassen einen Rückschluss über die erfolgte und die notwendige Unterstützung des Herzens durch die erfindungsgemäße Vorrichtung zu. Über die Sensoren am Herzen kann darüber hinaus auch die Herzfrequenz zur Frequenz der Unterstützungsvorrichtung angepasst werden. Dies kann aber zusätzlich oder alternativ auch durch ein EKG oder durch Koppelung mit einem Herzschrittmacher, insbesondere einem Zwei-Kammer-Herzschrittmacher realisiert werden. Diese Idee lässt sich, verallgemeinert, sogar auf eine Herzunterstützungsvorrichtung nach dem Oberbegriff des Anspruchs 1 anwenden.

Ein intrakorporaler Nachfülltank für Fluid, der über ein Nachfülltankventil mit dem Fluidkreislauf koppelbar ist, ermöglicht das Zuströmen von Fluid, insbesondere Druckfluid in den Fluidkreislauf, wenn zu wenig Fluid vorhanden sein sollte.

Der Nachfülltank ist vorzugsweise aus Metall (insbesondere Titan), Kunststoff oder Glas oder einer Sandwichkonstruktion aus diesen Materialien.

Auch das Nachfiilltankventil ist mit einer (insbesondere zentralen) Steuerung gekoppelt oder auch extrakorporal steuerbar. Beispielsweise lassen sich Daten wie der Maximaldruck in der Kammer von extrakorporal auslesen, sodass Rückschlüsse über die sich im Fluidkreislauf befindliche Fluidmenge gezogen werden können. Dann lässt sich beispielsweise durch den behandelnden Arzt die erforderliche fehlende Fluidmenge erhöhen. Dies erfolgt bevorzugt von extrakorporal über Sender-Empfänger-Einheiten, über die das Nachfülltankventil kurzzeitig geöffnet wird.

Dieses Nachfüllen erfolgt durch eine Steuerung vollautomatisch, indem bei Detektion von Fluidverlust selbsttätig das Nachfülltankventil in die offene Stellung angesteuert wird.

Es kann auch eine transkutan anschließbare Nachfüllstelle, insbesondere als passives Ventil ausgebildet, vorhanden sein, wobei die Ankoppelung des Nachfüllwerkzeugs z.B. über einen Schraub- oder Bajonettverschluss erfolgen kann.

Eine zentrale, subkutane Steuerung für die Pumpe und die Ventile kann einen Empfänger aufweisen sowie über den Empfänger umprogrammierbar ausgebildet sein. Somit lässt sich beispielsweise über einen geänderten Krankheitsverlauf die Arbeitsweise der erfindungsgemäßen Vorrichtung ändern. Wenn die entsprechende Steuerung auch einen Sender zum Auslesen von Daten besitzt, kann man von außen die Arbeitsweise der Herzunterstützungsvörrichtung oder auch, über die entsprechenden Sensoren, die Leistungsfähigkeit des Herzens ermitteln.

Eine extrakorporale, mit der subkutanen Steuerung kabellos gekoppelte Steuereinheit dient dazu, die Daten von der Vorrichtung abzugreifen und weiterzugeben. Eine Internetschnittstelle ermöglicht es, die Daten an einen zu behandelnden Arzt weiterzuleiten, möglicherweise sogar "live". Der Herzpatient muss also nicht den Arzt persönlich konsultieren, er kann sich mit dem Arzt verbinden. Der Arzt hat möglicherweise den Vorteil, dass Daten über einen längeren Zeitraum aufgenommen und an ihn weitergeleitet werden. Dies ermöglicht eine genauere Diagnose des Gesundheitszustandes sowie der Kontrolle der Arbeitsweise der erfindungsgemäßen Vorrichtung.

Ein erkranktes Herz kann sich auch im Lauf der Zeit verkleinern. Dies würde bedeuten, dass die Herzunterstützungsvorrichtung zu groß für das Herz wird oder nicht mehr optimal an es angepasst ist. Über stetig reduzierten Kammerdruck und/oder Blutdruck kann auf eine solche Verkleinerung des Herzens geschlossen werden. Die Daten werden über Drucksensoren zur Bestimmung des Kammerdrucks und/oder des Blutdrucks an die zentrale Steuerung für die Pumpe und für die Ventile geleitet, die die Daten auswertet. Vor allem die Variante, dass Blutdruck und Kammerdruck gemessen werden, ist besonders vorteilhaft. Die Ventile werden im Falle einer detektierten Herzverkleinerung von der Steuerung so betätigt, dass die Kammern weniger vollständig entleert und/oder bislang nicht benutzte Kämmern befüllt werden. Die Unterstützungsvorrichtung wird damit auf das sich verkleinernde Herz abgestimmt. Diese Idee ist bevorzugt an die Parallelschaltung der Kammern gekoppelt.

Eine Synchronisation der Vorrichtung mit dem Herzen kann insbesondere durch Koppelung der zentralen Steuerung für die Pumpe und für die Ventile mit einem Herzschrittmacher, insbesondere einem Zwei-Kammer-Herzschrittmacher, erreicht werden.

Auch oder zusätzlich könnte diese zentrale Steuerung für die Pumpe und die Ventile mit einem EKG gekoppelt sein.

Diese beiden Optionen dienen vor allem der Resynchronisation der Herztätigkeit.

Im Gegensatz zum Stand der Technik weist die erfindungsgemäße Herzunterstützungsvorrichtung ein eigensteifes und kein hochflexibles Trägerbauteil auf. Das Trägerbauteil gibt im Betrieb im Wesentlichen nicht nach, sodass sich die Kammern am Trägerbauteil abstützen können und nicht, wie bei einer flexiblen Manschette, der Innenquerschnitt der Manschette lediglich verringert wird. Durch das steife Trägerbauteil kann die Vorrichtung lokal gezielt gegen die Herzwand drücken.

Die Herzunterstützungsvorrichtung ist gemäß der bevorzugten Ausführungsform eine pneumatisch arbeitende Vorrichtung, d.h. die Kammern werden über Gas, insbesondere Luft inflatiert.

Darüber hinaus wird auch ein Verfahren zum Steuern einer erfindungsgemäßen Herzunterstützungsvorrichtung, die mit zumindest einer mit den Ventilen gekoppelten Steuerung versehen ist, offenbart. Diese Steuerung steuert die Ventile gruppenweise oder einzeln individuell an. Hierdurch ist es möglich, die natürliche Pumpbewegung des Herzens nachzubilden. Dazu kann es vorteilhaft sein, wenn die Kammern zeitlich versetzt zueinander befüllt/entleert werden.

Hierzu kann es gemäß der bevorzugten Ausführungsform vorteilhaft sein, zuerst die unteren Kammern zu befüllen, indem die zugeordneten Zulaufventile zuerst geöffnet werden. Zeitlich verzögert hierzu werden dann die weiter oben liegenden Kammern inflatiert. Auf diese Weise lässt sich die sogenannte Hebefunktion des Herzens in Richtung Aorta unterstützen.

Die Drehzahl der Pumpe sollte in Abhängigkeit von durch Durchflusssensoren oder Drucksensoren im Fluidkreislauf ermittelten Werten und/oder in Abhängigkeit von EKG-Werten bei Überschreiten oder Unterschreiten von vorgegebenen Werten verändert werden.

Zu betonen ist auch, dass generell die Druckwerte auch über Durchflusssensoren detektierbar sind.

Die bevorzugte Ausführungsform sieht zwar vor, dass die Herzunterstützungsvorrichtung mit einem becherförmigen Trägerbauteil, in den das Herz eingesetzt wird, ausgestattet ist und das Trägerbauteil im Perikard liegt. Alternativ hierzu kann das becherförmige Trägerbauteil aber auch außen um das Perikard herum angeordnet sein.

Weitere Merkmale und Vorteile der Erfindung ergeben sich aus der nachfolgenden Beschreibung und aus den nachfolgenden Zeichnungen, auf die Bezug genommen wird. In den Zeichnungen zeigen:
- Figur 1 eine schematische Ansicht der eingesetzten Herzunterstützungsvorrichtung,
- Figur 2 die Herzunterstützungsvorrichtung nach Figur 1 mit entferntem Trägerbauteil,
- Figur 3 die Herzunterstützungsvorrichtung nach der Erfindung in einer weiteren Ausführungsform,
- Figur 4 eine schematische Darstellung der Herzunterstützungsvorrichtung nach den vorhergehenden Figuren samt Steuerung und Pumpe,
- Figur 5 eine schematische Ansicht einer gegenüber Figur 4 leicht modifizierten Herzunterstützungsvorrichtung,
- Figuren 6a bis 6c schematische Ansichten der bei der erfindungsgemäßen Vorrichtung einsetzbaren Ventilschaltungen,
- Figur 7 eine schematische Ansicht eines Teils der Herzunterstützungsvorrichtung,
- Figur 8 eine Schnittansicht durch die Einheit aus Trägerbauteil und Kammern gemäß einer anderen Ausführungsform,
- Figur 9 eine schematische Darstellung, die die Herstellung des Trägerbauteils der erfindungsgemäßen Vorrichtung erläutert, und
- Figur 10 eine schematische Schnittansicht durch eine Spritzgussform zur Herstellung des Trägerbauteils der erfindungsgemäßen Herzunterstützungsvorrichtung.

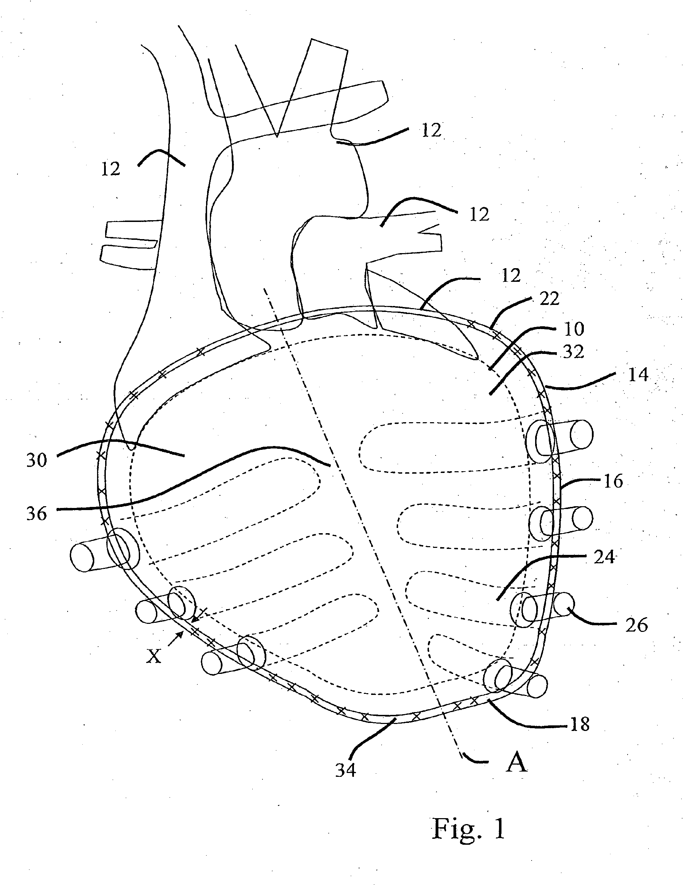

Figur 1 zeigt schematisch ein menschliches Herz 10, das mit unterbrochenen Linien dargestellt ist und von dem die bekannten Vorhöfe und großen Gefäß 12 zu- und abgehen.

Ein insuffizientes Herz kann seine Pumpfunktion nur unzureichend oder überhaupt nicht mehr ausführen. Zur Unterstützung der Pumpfunktion des Herzens 10 ist eine Herzunterstützungsvorrichtung vorhanden, die ein becherförmiges Trägerbauteil 14 mit einer geschlossen umlaufenden Seitenwand 16 und einem Bodenabschnitt 18 aufweist. Das Trägerbauteil 14 ist nach oben offen. Die entsprechende Öffnung trägt das Bezugszeichen 20. Durch sie hindurch erstrecken sich die Vorhöfe und großen Gefäße 12.

Am oberen Rand 38 des Trägerbauteils 14 verläuft eine axiale Halterung 22, z.B. eine angeschweißte oder angeformte Manschette, einwärts. Das Herz 10 ist in das Trägerbauteil 14, ohne dabei verletzt zu werden, von oben durch die Öffnung 20 eingesetzt und wird vom Trägerbauteil 14 umschlossen. Die axiale Halterung 22 ist zum leichteren Einführen des Herzens 10 vorzugsweise flexibel ausgeführt, kann also beim Einsetzen nach außen gedehnt werden. Zur verbesserten axialen Fixierung lässt sich die axiale Halterung 22 an der Perikardumschlagfalte oder anderen Punkten fixieren.

Auf der Innenseite des Trägerbauteils 14 sind an diesem mehrere benachbarte Kammern 24 vorgesehen, die vorzugsweise jeweils eine eigene Anschlussleitung 26 besitzen. Zur Verbesserung der Übersichtlichkeit ist nur eine der zahlreichen Kammern 24 und ihre zugeordnete Anschlussleitung 26 in Figur 1 mit einem Bezugszeichen versehen.

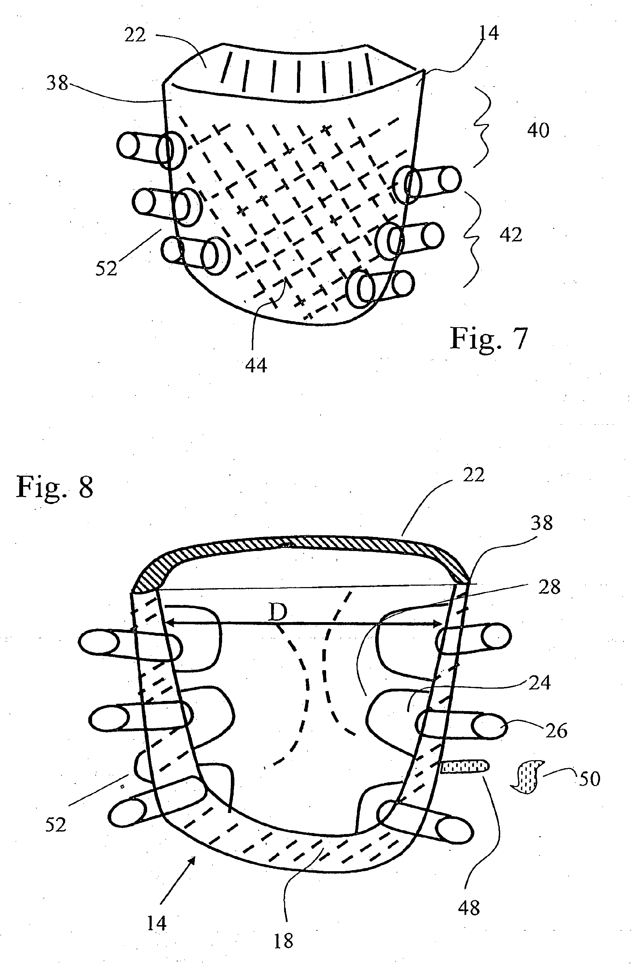

Figur 8 zeigt die Kammern 24 mit ihrer innenseitigen flexiblen, insbesondere elastischen Wand 28.

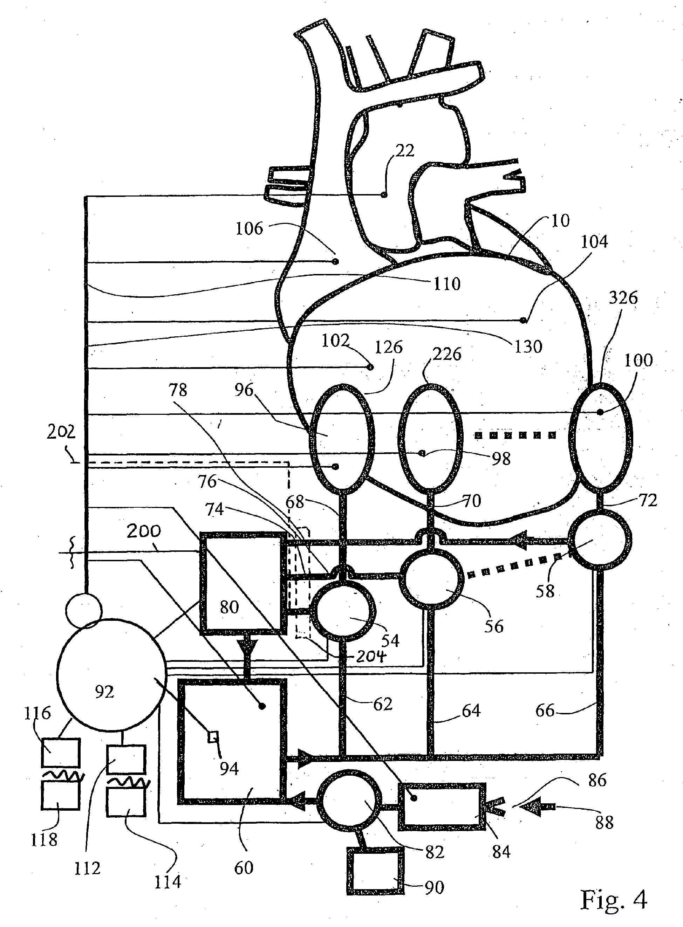

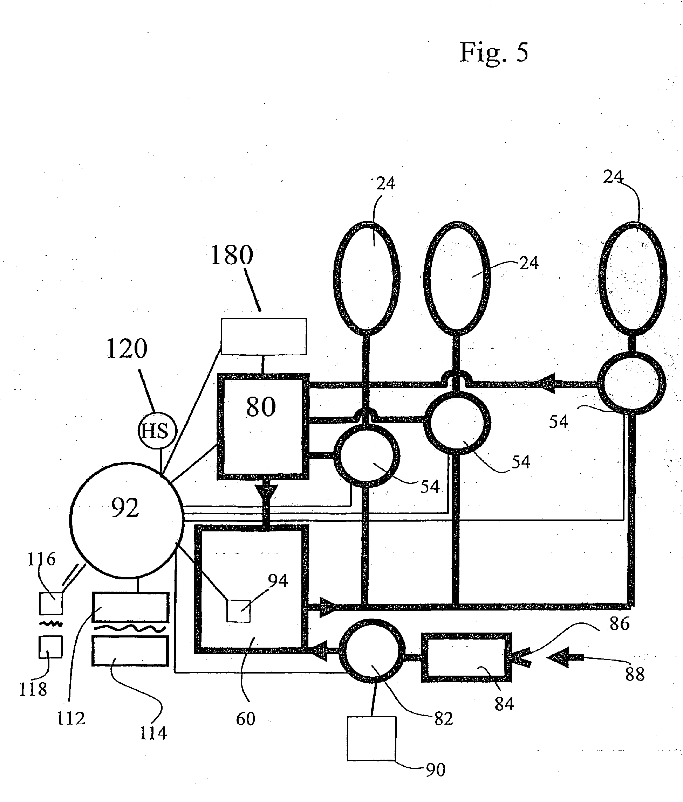

Die Kammern 24 sind Teil eines Fluidkreislaufes, der in den Figuren 4 und 5 exemplarisch dargestellt ist.

Die Kammern 24 können beispielsweise dadurch gebildet werden, dass eine Membran unmittelbar an das Trägerbauteil 14 innenseitig aufgeklebt oder aufgeschweißt wird, sodass die Kammern 24 innenseitig von der Membran und außenseitig durch das Trägerbauteil 14 begrenzt werden.

Alternativ hierzu können die Kammern 24 natürlich auch innenseitig durch eine eigene Wand definiert werden, welche am Trägerbauteil 14 befestigt wird.

Wie in Figur 1 zu sehen ist, verlaufen die Kammern 24 vorzugsweise, jedoch nicht zwingend, lediglich über einen Umfangsabschnitt; das heißt, sie laufen nicht geschlossen um. Insbesondere sind der linken Herzhälfte 30 und der rechten Herzhälfte 32 eigene Kammern 24 zugeordnet. Die Kammern 24 erstrecken sich, bezogen auf die von der Herzspitze 34 ausgehenden Achse A, die vorzugsweise entlang der Herzscheidewand verläuft, schräg aufwärts.

Die einander umfangsmäßig gegenüberliegenden Kammern 24, die den Herzhälften 30, 32 jeweils zugeordnet sind, überlappen einander vorzugsweise in Umfangsrichtung nicht, sodass ein streifenförmiger, axial verlaufender Abschnitt 36 entsteht, in dem keine Kammern 24 vorgesehen sind. Auch axial sind die Kammern 24, wie Figur 1 zeigt, voneinander getrennt und auch voneinander beabstandet.

Das Trägerbauteil 14 ist eigensteif und formstabil. Es wird so steif ausgebildet, dass es durch das pumpende Herz und die sich ausdehnenden Kammern nicht erwähnenswert deformiert wird. Aufgrund dieser Eigensteifigkeit ist es auch möglich, ein deformiertes Herz, das aufgrund seiner Deformation unzureichend schließende Herzklappen besitzt, in eine wieder zumindest annähernd normale Form zu bringen. Damit kann die natürliche Ventilschließfunktion verbessert werden.

Die Kammern 24 werden pulsierend mit Druckfluid befüllt und geleert und drücken zumindest in befülltem Zustand mit ihrer innenliegenden Wand 28 gegen das Herz 10, um es zu komprimieren und die Pumpfunktion zu unterstützen oder zu übernehmen.

Die Abstützung bei der Kompression des Herzens 10 erfolgt über das eigensteife, stabile Trägerbauteil 14. Die Kammern 24 können aufgrund des eigensteifen Trägerbauteils 14 an jeder beliebigen Stelle positioniert werden, ohne dass es damit zwangsläufig zu einer Gesamtkompression des Herzens 10 kommt, wie dies bei flexiblen Manschetten der Fall ist.

Das Trägerbauteil 14 hat eine Wanddicke von 1 bis 5 mm, die nicht konstant sein muss.

Figur 8 zeigt hierzu, etwas übertrieben dargestellt, dass die Wanddicke zum Bodenabschnitt 18 höher ist als im Bereich des oberen Randes 38, an dem die axiale Halterung 22 angebracht ist. Durch die unterschiedlichen Dicken ergibt sich eine unterschiedliche Steifigkeit des Trägerbauteils 14, wobei die Abschnitte unterschiedlicher Steifigkeiten insbesondere kontinuierlich ineinander übergehen. Im Bereich des oberen Randes 38 hat das Trägerbauteil 14 vorzugsweise eine geringere Steifigkeit als im Bereich des herzspitzenseitigen Bodenabschnitts 18. Damit lässt sich der obere Rand 38 etwas weicher ausbilden, was die Herzbelastung an dieser Stelle verringert.

Die Steifigkeit des Trägerbauteils 14 kann in axialer und/oder Umfangsrichtung unterschiedlich sein, um optimal auf das Herz 10 individuell abgestimmt zu sein.

Die unterschiedliche Eigensteifigkeit lässt sich auch durch andere Maßnahmen erreichen, beispielsweise durch unterschiedliche Materialien, die ineinander übergehen, oder durch den unterschiedlichen Anteil an Verstärkungsmaterial, welches im Trägerbauteil eingebettet sein kann. Dies wird anhand von Figur 7 später noch näher erläutert.

Die Steifigkeit ist gemäß einer Ausführungsform so gewählt, dass während des Betriebes der Vorrichtung das Trägerbauteil 14 außenseitig um maximal 3 mm, vorzugsweise maximal 1 mm lokal ausbaucht, wie anhand von Figur 1 gezeigt ist und wo X die Ausbauchungsstrecke angibt.

Als Herstellungsmaterialien für das Trägerbauteil 14 kommen verschiedene Werkstoffe infrage, insbesondere jedoch Kunststoff, z.B. Polyamid, PTFE beschichtetes Polyamid oder PEEK.

Alternativ hierzu kann das Trägerbauteil 14 natürlich auch aus Metall, insbesondere Titan oder Nitinol, bestehen. Aber auch Verbundbauteile aus Kunststoff und Metallverstärkungen sind möglich.

Beim Einsatz von Metall können Formgedächtnislegierungen besondere Vorteile bringen.

In Figur 7 ist dargestellt, dass das Trägerbauteil 14 einen oberen, weicheren Bereich 40 und einen unteren, steiferen Bereich 42 besitzt.

Vorzugsweise sind beide Bereiche 40, 42 formstabil bezogen auf den normalen Druck während des Betriebs der Vorrichtung. Im Kunststoff des Trägerbauteils 14 sind Metallverstärkungen 44 vollständig eingebettet, zum Beispiel durch Umspritzen derselben. Die Metallverstärkungen 44, hier aus einer Formgedächtnislegierung, sind gitterförmig ausgeführt, um ihre Einbettung im Kunststoff zu verbessern und eine gewisse Flexibilität zu ermöglichen.

Im Bereich 40 ist flächenbezogen weniger Verstärkungsmaterial vorhanden, das heißt weniger Metallverstärkungen, um den Bereich 40 weicher zu gestalten als den darunter liegenden Bereich 42.

Für die normale Belastung während des Betriebes sollte, wie mehrfach erwähnt, das Trägerbauteil 14 eigensteif sein. Bei höheren Belastungen jedoch, zum Beispiel bei einer Herzmassage oder bei einem Schlag gegen den Brustkorb, ist das Trägerbauteil 14 flexibel genug, um ein radiales, elastisches Zusammendrücken zu ermöglichen. Im nicht eingebauten Zustand ist die Elastizität so groß, dass sich das Trägerbauteil 14, ohne sich plastisch zu verformen oder zu brechen, um mindestens 75 % gegenüber dem unbelasteten Zustand deformieren lässt. In Figur 8 ist hierzu der Abstand D benachbarter Wandabschnitte im unbelasteten Zustand dargestellt sowie mit unterbrochenen Linien, wenn das Trägerbauteil 14 komprimiert wird. Dieser Abstand D kann auf mindestens 75 % reduziert werden, ohne dass es zu einer Zerstörung des Trägerbauteils 14 kommt. Die Elastizität des Trägerbauteils 14 kann sogar so weit gehen, dass sich die gegenüberliegenden Wandabschnitte berühren (siehe Figur 8), wobei hierbei natürlich unter Berühren auch zu verstehen ist, dass die innenliegenden Wände der nicht aufgeblasenen Kammern 24 einander berühren.

Natürlich muss die Flexibilität des Trägerbauteils 14 nicht in jedem Abschnitt, insbesondere nicht im Bereich des Bodenabschnitts 18 vorhanden sein. Es reicht aus, wenn das obere Drittel, insbesondere die obere Hälfte, die zuvor erwähnte Elastizität besitzt.

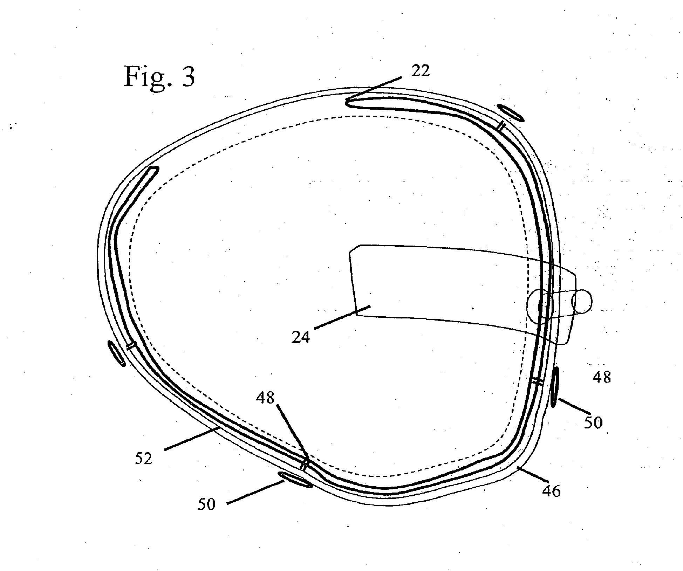

Zur Lagesicherung des Trägerbauteils 14 im Körper kann dieses in den Herzbeutel 46 (siehe Figur 3) eingesetzt werden. Hierdurch ist schon eine erste Lagefixierung erreicht.

Zusätzlich lassen sich auch noch Befestigungen vorsehen, beispielsweise an der Außenseite des Trägerbauteils 14 angebrachte, insbesondere angeformte Befestigungsfortsätze 48, welche durch kleine Öffnungen im Herzbeutel 46 hindurchragen. Von außen wird ein Verschluss 50 auf das freie Ende des Befestigungsfortsatzes 48 aufgesetzt, um ein Herausgleiten des Befestigungsfortsatzes 48 zu verhindern. Dieser Verschluss 50 ist insbesondere ein Druckknopf, der an einem entsprechend geformten Ende des Befestigungsfortsatzes 48 aufgesetzt wird. Wie in Figur 3 zu sehen, können mehrere Befestigungsfortsätze 48 an beliebigen Stellen angebracht werden.

Alternativ oder zusätzlich hierzu wird das Trägerbauteil 14 am Herzbeutel 46, an der Perikardumschlagfalte an der Aorta oder am Ligamentum pulmonale angenäht. Eine am Trägerbauteil 14 vorgesehene, angeformte Fadenhalterung 52 ist insbesondere in den Figuren 3 und 8 gut zu erkennen.

Um die Reibung auf der Herzaußenseite zu reduzieren, besitzt die Einheit aus Trägerbauteil 14 und Kammern 24 innenseitig eine Wandung oder eine Beschichtung aus PEEK oder PTFE oder anderen friktionsarmen Beschichtungen/Materialien. Hierzu lässt sich beispielsweise eine zusätzliche komplette Wand, die wie ein Beutel innerhalb des Trägerbauteils 14 verläuft, ausbilden. Optional lassen sich aber auch die innenseitigen Wände 28 entsprechend ausführen.

Die axiale Höhe des Trägerbauteils 14 von der Herzspitze 34 aus gemessen sollte so gewählt sein, dass der obere Rand 38 etwa 10 bis 20 mm unterhalb der sogenannten Herzklappenebene liegt. Eine andere Definition in diesem Zusammenhang, die ebenfalls eine Beeinflussung der Klappen reduzieren oder ausschließen soll, besteht darin, keine Kammern 24 bis in die Klappenebene ragen zu lassen. Die Kammern 24 sollten dann ebenfalls 10 bis 20 mm unterhalb der Klappenebene enden.

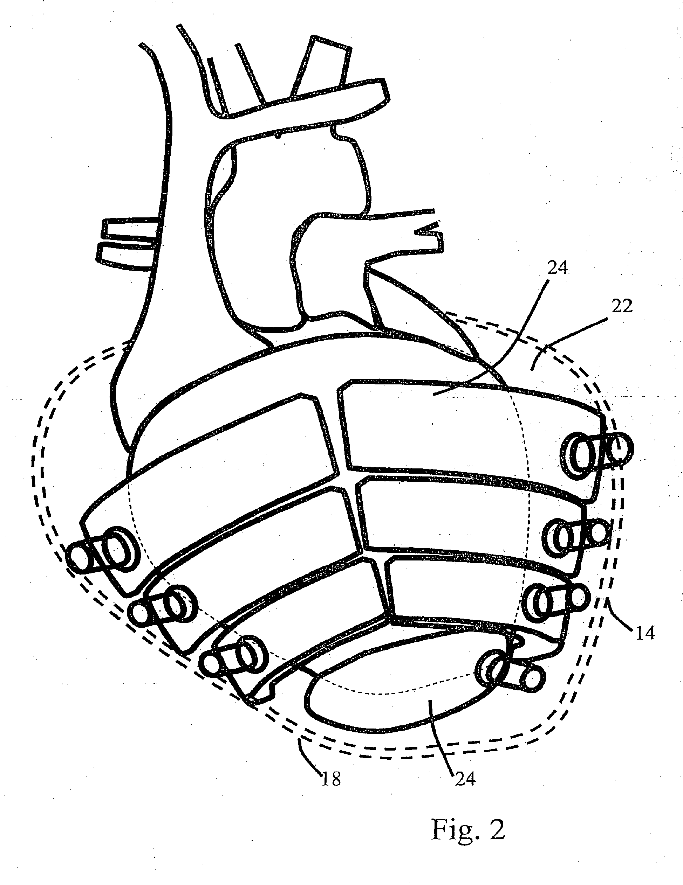

Figur 2 gibt eine bessere Darstellung der einzelnen Kammern. Im Gegensatz zu Figur 1 ist die unterste Kammer 24' großvolumiger ausgeführt und deckt den Bodenabschnitt 18 großteils ab. Über diese Kammer 24' lässt sich die axiale Innenhöhe der Einheit aus Trägerbauteil 14 und Kammern 24 etwas variieren und an das Herzvolumen anpassen. Natürlich kann auch über die Kammer 24' die Pumpfunktion unterstützt werden.

Figur 3 soll verdeutlichen, dass die Kammern 24 natürlich nicht die annähernd gesamte Innenseite des Trägerbauteils 14 bedecken müssen. Vielmehr ist es im Hinblick auf die möglicherweise lokale Schwäche des Patientenherzens wesentlich besser, an einzelnen, wenigen Stellen Kammern 24 vorzusehen. Es können also einige oder viele Kammern 24 vorgesehen sein, wobei natürlich vorzugsweise die Vorrichtung mit zahlreichen Kammern 24 ausgeführt wird.

In Figur 4 ist eine Übersicht über die gesamte Herzunterstützungsvorrichtung zu sehen, bei der die Einheit aus Trägerbauteil 14 und Kammern symbolisch durch drei Kammern 126, 226 und 336 angegeben ist. Natürlich sind auch mehr als drei Kammern 126, 226 oder 326 entsprechend in die Gesamtvorrichtung integrierbar. Jede Kammer 126, 226, 326 steht über ein eigenes, nur ihr zugeordnetes Mehrwegventil 54 bis 58 mit einem Druckfluidspeicher 60 in Strömungsverbindung. Vom Druckfluidspeicher 60 verlaufen Leitungen 62 bis 66 zu den Ventilen 54 bis 58. Diese Leitungen 62 bis 66 sind Zuströmleitungen. Leitungen 68 bis 72 zwischen den Ventilen 54 bis 58 zu den Kammern 26 bis 326 sind kombinierte Zu- und Abströmleitungen.

Von den Ventilen 54 bis 58, die als kombinierte Zuström- und Abströmventile dienen, verlaufen Abströmleitungen 74 bis 78 zu einer Fluidpumpe 80, die Fluid in den Speicher 60 pumpt.

Natürlich können anstatt der kombinierten Zu- und Abströmleitungen 68 bis 72 auch separate Zu- und Abströmleitungen zu und von den Kammern 126 bis 326 weggehen.

Die Pumpe 80 arbeitet vorzugsweise permanent und erzeugt einen permanenten Druck im Speicher 60. Der Speicher 60 wiederum steht über ein Nachfülltankventil 82 mit einem intrakorporalen Fluidnachfülltank 84 in Strömungsverbindung. Der Fluidnachfülltank 84 enthält Fluid (vorzugsweise unter Druck stehend), das in den zuvor erläuterten Fluidkreislauf über das temporär geöffnete Ventil 82 zuströmt, wenn zu wenig Fluid im Kreislauf enthalten ist.

Der Nachfülltank ist vorzugsweise aus Metall (insbesondere Titan), Kunststoff oder Glas oder einer Sandwichkonstruktion aus diesen Materialien.

Zusätzlich zu dem Fluidnachfülltank 84 kann eine transkutan anschließbare Nachfüllschnittstelle 86 vorgesehen sein, die insbesondere als passives Ventil ausgebildet ist und zum Beispiel über eine Spritze 88 von außen zugänglich ist. Hierüber kann bei mangelndem Fluid im Kreislauf von außen Fluid nachgefüllt werden. Die Schnittstelle 86 kann auch unmittelbar am Speicher 60 angekoppelt sein.

Die Ankoppelung des Nachfüllwerkzeugs (hier Spritze) kann z.B. über einen Dreh(d.h. Schraub-) oder Bajonettverschluss erfolgen.

Das Nachfülltankventil 82 ist entweder über eine eigene Steuerung 90 oder über eine zentrale Steuerung 92 ansteuerbar. Die Steuerung 90 kann beispielsweise auch von extrakorporal über Senderund Empfänger-Einheiten angesteuert werden, um das Nachfülltankventil 82 temporär zu öffnen. Auch die Menge des zuströmenden Fluids wird durch die Öffnungszeit des Nachfülltankventil 82 gesteuert.

Die Steuerung 90 oder 92, die für das Nachfülltankventil 82 zuständig ist, ist so ausgebildet, dass sie bei Fluidverlust im Fluidkreislauf selbsttätig das Nachfülltankventil 82 ansteuert. Aber, wie bereits erläutert, dieses Steuern kann auch von extrakorporal erfolgen.

Ein Drucksensor 94 im Speicher 60, der mit der Steuerung 92 verbunden ist, gibt Auskunft über den Druck im Speicher 60. Die im Folgenden als zentrale Steuerung beschriebene Steuerung 92 steht mit der Pumpe 80, den einzelnen Ventilen 54 bis 58, gegebenenfalls dem Nachfülltankventil 82, sowie mit Sensoren 96 bis 100, die in den Kammern 126 bis 326 untergebracht sind, in Verbindung. Hierüber lassen sich Aussagen über den Druck in den Kammern 126 bis 326 machen. Weitere Drucksensoren 102, 104 erfassen den Druck in den beiden Herzkammern sowie Drucksensoren 106 bis 108 in der Vene bzw. der Aorta.

Zusätzlich oder alternativ zu den Drucksensoren 102 bis 104 kann die zentrale Steuerung 92 auch mit einem EKG-Sensor 130 gekoppelt sein und/oder mit einem Herzschrittmacher, insbesondere einem Zwei-Kammer-Herzschrittmacher 1 10. Die Pumpe 80 kann direkt oder über die zentrale Steuerung 92 mit einem Energiespeicher 112, insbesondere einem Akku, verbunden sein. Dieser Energiespeicher 112 wird insbesondere, ohne durch die Haut Leitungen führen zu müssen, von außen über einen magnetischen oder elektrischen Sender 114 permanent oder temporär aufgeladen. Die zentrale Steuerung 92 kann von außen, ebenfalls über Sender-Empfängereinheit 1 16, 1 18 kontrolliert werden oder auch umprogrammiert werden. Darüber hinaus kann die außerhalb des Körpers vorgesehene Sender-Empfängereinheit 1 18 auch mit einer Internetschnittstelle, insbesondere über Wireless-LAN, gekoppelt sein. So ist es möglich, den Patienten sozusagen von zuhause aus mit einem Arzt zu verbinden, sodass dieser die Werte des Patienten auslesen und gegebenenfalls die zentrale Steuerung 92 umprogrammieren kann.

Im Betrieb sorgt die vorzugsweise mit einem bürstenlösen Antrieb ausgeführte Pumpe 80 für einen permanenten Überdruck im Druckfluidspeicher 60. Die zentrale Steuerung 92 steuert die Ventile 54 bis 58 einzeln an, sodass Druckfluid aus dem Speicher 80 die Kammern 126 bis 326 pulsierend aufbläst und damit das Herz 10 verengt. Die Kammern 126 bis 326 werden unabhängig einzeln oder gruppenweise über die Ventile 54 bis 58 (gemäß Figur 4 einzeln) angesteuert. Nach dem Zusammendrücken des Herzens 10 erfolgt druck- und/oder EKGgesteuert eine Umsteuerung der Ventile 54 bis 58, sodass die Kammern 126 bis 326 mit der Pumpe 80 strömungsverbunden sind und abrupt geleert werden. Das Herz 10 kann sich so wieder ausdehnen.

Das Ausmaß der Kompression des Herzens 10 kann von den durch die Sensoren ermittelten Druck- oder Strömungswerten abhängig sein.

Die erfindungsgemäße Vorrichtung lässt sich, wie gezeigt, als geschlossener Kreislauf ausführen, der komplett intrakorporal liegt, oder der teilweise auch extrakorporal positioniert ist (z.B. Pumpe oder Energiespeicher).

Eine weitere Ausführungsform der Erfindung sieht vor, die Vorrichtung als offenes System zu betreiben. Dies bedeutet, dass die Vorrichtung nach den Kammern über eine aus dem Körper herausragende Leitung die Druckluft nach außen ablässt. Falls vorhanden, können die Abströmventile noch intrakorporal liegen. Die intra- oder extrakorporal liegende Pumpe saugt Luft aus der Atmosphäre an. Mit unterbrochenen Linien ist in Figur 4 hierzu eine zur Atmosphäre führende Ansaugleitung 200 dargestellt. Die Leitungen 74, 76, 78 würden hier nicht an die Pumpe 80 anschließen, sondern würden über eine Sammelleitung 202 nach außerhalb des Körpers führen.

Zusätzlich oder alternativ zum Druckspeicher 60 kann auch stromaufwärts der Pumpe eine Druckfluidsammelkammer 204 (unterbrochene Linie in Figur 4) vorhanden sein, in die die von den einzelnen Kammern kommenden Leitungen münden. Die Druckfluidsammelkammer dient als Fluidzwischenspeicher.

Die Herzunterstützungsvorrichtung ist mit einer Notfallsensorik ausgestattet, die die Sensoren 96 bis 100 nutzt. Sobald die Steuerung 92 einen übermäßigen Druckabfall in einer der Kammern 126 bis 326 detektiert, wird das entsprechende Ventil 54 bis 58 permanent geschlossen, sodass über die defekte Kammer kein Fluid mehr ausströmen kann.

Die Steuerung 92 ist ferner so ausgeführt, dass sie bei Unterschreiten/Überschreiten eines vorgegebenen Druckminimums bzw. Druckmaximums im Druckspeicher oder bei erhöhter Pulsfrequenz die Drehzahl der Pumpe 80 verändert.

Das durch die Vorrichtung unterstützte Herz 10 kann unter Umständen im Laufe der Jahre kleiner werden. Dadurch ist die Unterstützungsfunktion der Vorrichtung möglicherweise geringer und/oder der Sitz der Einheit aus Trägerbauteil 14 und Kammern 126 bis 326 kann sich verschlechtern. Sobald dieses Verkleinern des Herzens 10 detektiert wird, kann die Steuerung 92 die Ventile 54 bis 58 so ansteuern, dass die Kammern 126 bis 326 weniger vollständig entleert werden, das heißt, dass sie stetig an der Herzwand anliegen. Zudem oder alternativ hierzu können bislang nicht benutzte Kammern wie beispielsweise die in Figur 2 gezeigte Kammer 24' befüllt oder stärker befüllt werden.

Eine Möglichkeit, um ein sich verkleinerndes Herz zu detektieren, besteht darin, dass dies aus einem stetig reduzierten Kammerdruck und/oder Blutdruck rückgeschlossen wird. Die Kammern 126 bis 326 müssen nicht gleichzeitig befüllt werden. Vielmehr ist es gegebenenfalls vorteilhaft, sie zeitlich verzögert zu befüllen und wieder zu entleeren. So können beispielsweise die (mit Bezug auf Figur 2) linken Kammern 24 zeitlich etwas vor den rechten Kammern 24 befüllt werden, damit die linke Herzhälfte vor der rechten Herzhälfte einen äußeren Druck erfährt.

Ferner ist es möglich, die Kammern 24 von der Herzspitze 18 aus zeitlich etwas gestaffelt zu befüllen, sodass die obersten Kammern 24 zuletzt befüllt werden. Der zeitlich gestaffelte Aufblasvorgang von der untersten bis zur obersten Kammer 24 soll die sogenannte Hebefunktion des Herzens in Richtung Aorta sicherstellen.

Der Druck in den Kammern 24, 126 bis 326 oder in den Herzhälften gibt auch eine Aussage über die Funktion und die Leistungsfähigkeit des Herzens. Durch über Drucksensoren ermittelte Druckwerte und über Druckverläufe lässt sich eine Funktionsevaluation des Herzens errechnen und gegebenenfalls dreidimensional darstellen. Ein gesundes Herz hat weniger Gegendruck, sodass auch weniger Fluid in die Kammern 24, 126 bis 326 eingeleitet werden muss. Sollte sich das Herz bezüglich seiner Leistungsfähigkeit erholen, so wird über die Steuerung 92 ebenfalls ein geringerer Druck in den Kammern 24, 126 bis 326 erzeugt.

Wie bereits zuvor anhand der Figuren 1 bis 3 erläutert, kann die Größe, Anzahl und Position sowie das Volumen der Kammern 24, 126 bis 326 variieren, um die Vorrichtung auf die Erkrankung des Herzens und die Größe des Herzens abzustimmen.

Die Ausführungsform nach Figur 5 entspricht im Wesentlichen der nach Figur 4, sodass im Folgenden nur noch auf die Unterschiede eingegangen werden muss.

Bei dieser Ausführungsform ist zusätzlich zur Pumpe 80 auch noch eine mit ihr gekoppelte Notlaufpumpe 180 gekoppelt. Sobald die Pumpe 80 ausfällt, wird über die Steuerung 92 die Notlaufpumpe 180 zugeschaltet, die dann für den erforderlichen Druck im Fluid sorgt. Zusätzlich oder alternativ zu den bisherigen Sensoren sind auch noch sogenannte Triggersensoren 120 vorgesehen, über die eine Abstimmung zwischen der Herzfunktion und der Pumpfunktion der Vorrichtung erfolgen kann.

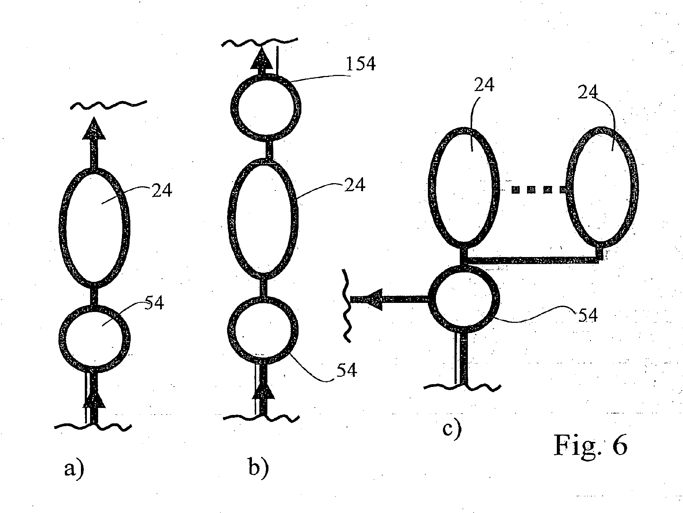

Figur 6 zeigt verschiedene weitere Ausführungsformen, wie die Kammern 24, 126 bis 326 angesteuert werden.

Bei der Ausführungsform nach Figur 6a ist eine permanente Abströmung vorgesehen, sodass nur das Ventil 54 für den Druckaufbau in der Kammer 24 verantwortlich ist.

Bei der Ausführungsform nach Figur 6b sind getrennte Einlass- und Auslassventile 54, 154 vorgesehen.

Bei der Ausführungsform nach Figur 6c wiederum werden mehrere Kammern 24 über ein gemeinsames Ventil, hier ein kombiniertes Zu- und Abströmventil 54, angesteuert.

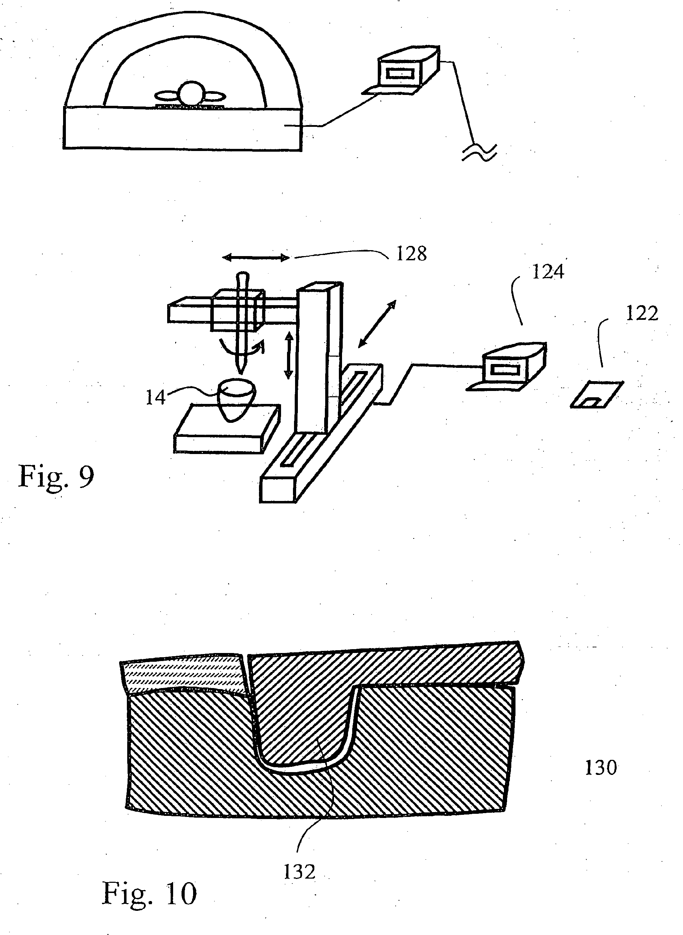

Im Folgenden wir die Herstellung des Trägerbauteils 14 näher erläutert. Das Trägerbauteil 14 wird an die Form des Herzens 10 des Patienten speziell angepasst und individuell gefertigt. Diese Fertigung erfolgt als dreidimensionale maßgeschneiderte Fertigung. Hierzu werden durch ein MRT- und/oder CT-Scanverfahren (siehe Figur 9) Daten des Herzens gewonnen, die eine dreidimensionale Herzaußenform oder Herzbeutelaußenform direkt oder indirekt wiedergeben. Indirekt bedeutet, dass über das MRT oder CT ermittelte Daten die entsprechenden Daten für die 3-D-Form des Herzens errechnet werden (Oberflächenrekonstruktion; Sulfate Rendering). Diese Daten werden direkt oder über einen Datenspeicher 122 in die Steuerung 124 eines Bearbeitungszentrums 128 eingegeben. Im Bearbeitungszentrum 128 werden diese Daten vollautomatisch umgesetzt, um entweder das Herz nachzubilden oder gleich direkt das Trägerbauteil 14 herzustellen. Das Bearbeitungszentrum 126 ist beispielsweise ein Zentrum zum Lasersintern, Durchführen einer Stereolithografie oder dergleichen. Alternativ kann es natürlich auch ein spanendes Bearbeitungszentrum sein.

In diesem Zusammenhang wäre es beispielsweise möglich, dass über die gewonnene 3-D-Form ein Trägerbauteil 14 aus einer Vielzahl vorgefertigter Trägerbauteile ausgesucht wird, welches eine geringere Nachbearbeitung als die anderen erfordert. Das Bearbeitungszentrum 128 könnte beispielsweise ein Fräsbearbeitungszentrum sein, über das dann die Feinabstimmung erfolgt.

Als weitere Alternative wäre es möglich, dass das Bearbeitungszentrum 128 ein oder mehrere Elemente einer Spritzgussform 130 (siehe Figur 10) anfertigt. Über diese Spritzgussform 130 wird dann das Trägerbauteil 14 gegossen (siehe Figur 10), bei der eine Patrize 132 individuell hergestellt worden ist. Durch die vom Herzen durch das CT- oder MRT-Verfahren gewonnenen Daten, aus denen sich die 3-D-Form des Herzens ermitteln lässt, können auch die Größe und die Lage der Kammern 24, 126 bis 326 auf die Situation exakt abgestimmt werden.

Eine Kombination der einzelnen, individuell ansteuerbaren Kammern 24, 126 bis 326 mit einem Zwei-Kammer-Herzschrittmacher-System dient der Resynchronisation der Herztätigkeit.

Anstatt im Inneren des Herzbeutels kann das Trägerbauteil 14 auch außen am Herzbeutel sitzen und auch diesen umgeben. Alle vorgenannten Merkmale sind entsprechend auch bei dieser Alternative verwendbar.

Das gerade beschriebene Verfahren wird ebenso für die Herstellung eines Implantat angewandt, das ein inneres Organ oder eine innere Organstruktur zumindest teilweise ersetzt oder an dieses angrenzend implantiert wird, um das Organ zu unterstützen. Dieses Implantat wird individuell auf Basis von durch ein bildgebendes Verfahren gewonnenen, die dreidimensionale Organform oder das durch das Implantat ersetzte Körperteil wiedergebenden Daten gefertigt wird. Das Implantat ersetzt nicht ein hartes Körperteil wie einen Zahn oder einen Knochen, sondern ersetzt oder unterstützt ein weiches Körperteil im Inneren des Körpers. Demzufolge wird durch das bildgebende Verfahren die Kontur eines weichen Körperteiles im Körperinneren ermittelt, um das Implantat individuell herstellen zu können.

Das Implantat ist insbesondere die Aorta, worunter natürlich auch ein Aortaabschnitt fällt (aszendens, Bogen, deszendens), oder eine Herzklappe oder ein das Zwerchfell oder die Lunge stützendes an diese angrenzendes Trägerbauteil.

Besonders bevorzugt wird bei diesem Verfahren Lasersintern angewandt, wobei die verwendeten Materialien die zuvor und nachfolgend erwähnten Materialien oder Sandwichmaterialien sind.

Die Herzunterstützungsvorrichtung ist bevorzugt eine pneumatisch arbeitende Vorrichtung, d.h. die Kammern werden über Gas, insbesondere Luft inflatiert.

## Patentansprüche

1. Herzunterstützungsvorrichtung mit einem das Herz umschließenden Trägerbauteil, an dem innenseitig mehrere benachbarte inflatierbare und deflatierbare Kammern (24; 126, 226, 326) vorgesehen sind, über die eine innenseitige Wand (28) einwärts verschiebbar ist, wobei die Kammern (24; 126, 226, 326) über Leitungen mit wenigstens einer Pumpe (80) in Strömungsverbindung stehen, wobei mehrere zuströmseitige Zuströmventile zwischen Pumpe (80) und Kammern (24; 126, 226, 326) sind, wobei zumindest einzelne Kammern (24; 126, 226, 326) einem eigenen Zuströmventil oder Gruppen von Kammern (24; 126, 226, 326) eigenen Zuströmventilen zugeordnet sind, um die Kammern (24; 126, 226, 326) unabhängig einzeln bzw. gruppenweise anzusteuern,
wobei ein intrakorporaler Fluidnachfülltank (84) über ein intrakorporales Nachfülltankventil (82) mit dem Fluidkreislauf koppelbar ist, um bei geöffnetem Nachfülltankventil (82) Fluid in den Fluidkreislauf strömen zu lassen,
**dadurch gekennzeichnet, dass** das Nachfülltankventil (82) mit einer zentralen Steuerung (60) gekoppelt oder extrakorporal steuerbar ist und die zentrale Steuerung (60) so ausgebildet ist, dass sie bei Fluidverlust im Fluidkreislauf selbsttätig das Nachfülltankventil (82) in die offene Stellung ansteuert.

2. Herzunterstützungsvorrichtung nach Anspruch 1, **dadurch gekennzeichnet, dass** zwischen Pumpe (80) und Kammern (24; 126, 226, 326) zuströmseitig wenigstens ein Druckspeicher (60) angeordnet ist, in den die Pumpe (80) Druckfluid einspeist, wobei die Ventile stromabwärts des Druckspeichers (60) angeordnet sind, und/oder dass zwischen Kammern (24; 126, 226, 326) und Pumpe abströmseitig wenigstens eine Druckfluidsammelkammer (204) angeordnet ist, in die sämtliche von den Kammern (24; 126, 226, 326) Richtung Pumpe (80) führenden Leitungen münden.

3. Herzunterstützungsvorrichtung nach Anspruch 2, **dadurch gekennzeichnet, dass** ein den Druck im Druckspeicher (60) ermittelnder Drucksensor (94) vorgesehen ist.

4. Herzunterstützungsvorrichtung nach Anspruch 3, **dadurch gekennzeichnet, dass** der Drucksensor (94) mit einer Steuerung (60) der Pumpe (80) gekoppelt ist, die so ausgebildet ist, dass bei Unterschreiten/Überschreiten eines vorgegebenen Druckminimums bzw. Druckmaximums im Druckspeicher (60) die Drehzahl der Pumpe (80) verändert wird.

5. Herzunterstützungsvorrichtung nach einem der vorhergehenden Ansprüche, **dadurch gekennzeichnet, dass** von jeder Kammer (24; 126, 226, 326) eine eigene Zuström- und/oder eine eigene Abströmleitung ausgeht.

6. Herzunterstützungsvorrichtung nach Anspruch 5, **dadurch gekennzeichnet, dass** abströmseitig die Kammern (24; 126, 226, 326) unmittelbar mit der Pumpe (80) strömungsverbunden sind und/oder dass die Vorrichtung als offenes System ausgeführt ist, bei dem die Pumpe (80) Luft aus der Atmosphäre ansaugt und die Luft aus den Kammern (24; 126, 226, 326) in die Atmosphäre abgeblasen wird.

7. Herzunterstützungsvorrichtung nach einem der vorhergehenden Ansprüche, **dadurch gekennzeichnet, dass** eine Steuerung (60) der Ventile (54, 56, 58) und eine damit gekoppelte Notfallsensorik vorgesehen sind, wobei die Steuerung (60) so ausgebildet ist, dass sie bei Detektion einer Leckage einer Kammer (54, 56, 58) die der defekten Kammer zugeordneten Ventile (54, 56, 58) bleibend schließt.

8. Herzunterstützungsvorrichtung nach einem der vorhergehenden Ansprüche, **dadurch gekennzeichnet, dass** zusätzlich zur Pumpe (80) eine Notlaufpumpe (180) sowie eine Steuerung (60) der Pumpen (80, 180) vorgesehen ist, wobei die Steuerung (60) so ausgebildet ist, dass sie bei Detektion eines Defekts der laufenden Pumpe (80) die Notlaufpumpe (180) aktiviert.

9. Herzunterstützungsvorrichtung nach einem der vorhergehenden Ansprüche, **dadurch gekennzeichnet, dass** herzseitige Drucksensoren (102, 104, 106, 108) mit einer Steuerung (60) für Ventile (54, 56, 58) und/oder die Pumpe (80) gekoppelt sind.

10. Herzunterstützungsvorrichtung nach einem der vorhergehenden Ansprüche, wobei der Nachfülltank (84) aus Metall, insbesondere Titan, Glas oder Kunststoff oder einer Sandwichkonstruktion aus diesen Materialien besteht.

11. Herzunterstützungsvorrichtung nach einem der vorhergehenden Ansprüche, **dadurch gekennzeichnet, dass** der Fluidkreislauf eine transkutan anschließbare Nachfüllschnittstelle (86), die insbesondere als passives Ventil ausgebildet ist, aufweist, wobei die Nachfüllschnittstelle vorzugsweise über einen Schraub- oder Bajonettverschluss mit einem von außen anschließbaren Nachfüllwerkzeug lösbar gekoppelt werden kann.

12. Herzunterstützungsvorrichtung nach einem der vorhergehenden Ansprüche, **dadurch gekennzeichnet, dass** eine zentrale, subkutane Steuerung (60) für die Pumpe (80) und die Ventile (54, 56, 58) vorgesehen ist, die einen Empfänger (116) aufweist sowie über den Empfänger (1 16) umprogrammierbar ausgebildet ist und/oder die einen Sender (116) zum Auslesen von Daten aufweist.

13. Herzunterstützungsvorrichtung nach Anspruch 12, **gekennzeichnet durch** eine extrakorporale, mit der subkutanen Steuerung (60) kabellos koppelbare Steuereinheit (118), die eine Internetschnittstelle aufweist.

14. Herzunterstützüngsvorrichtung nach einem der vorhergehenden Ansprüche, **dadurch gekennzeichnet, dass** eine zentrale Steuerung (60) für die Pumpe (80) und die Ventile (54, 56, 58) vorgesehen ist, die mit Drucksensoren (96 - 108) zum Bestimmen des Kammerdrucks und/oder des Blutdrucks gekoppelt ist, wobei die Steuerung (60) so ausgebildet ist, dass sie bei einem stetig reduzierten Kammerdruck und/oder Blutdruck die Ventile (54, 56, 58) so ansteuert, dass die Kammern (24; 126, 226, 326) weniger vollständig entleert und/oder bislang nicht benutzte Kammern (24; 126, 226, 326) befüllt werden.

## Claims

1. A cardiac assistance device having a supporting component which surrounds the heart and on the inside of which a plurality of adjacent inflatable and deflatable chambers (24, 126, 226, 326) are provided, via which an inside wall (28) can be displaced inward, wherein the chambers (24, 126, 226, 326) have a fluidic connection to at least one pump (80) via lines, wherein a plurality of supply valves are on the supply side between pump (80) and chambers (24, 126, 226, 326), wherein at least individual chambers (24, 126, 226, 326) are assigned a dedicated supply valve, or groups of chambers (24, 126, 226, 326) are assigned dedicated supply valves, in order to control the chambers (24, 126, 226, 326) independently, either individually or in groups,
wherein an intracorporeal fluid refill tank (84), which can be coupled to the fluid circuit via an intracorporeal refill tank valve (82), in order to allow fluid to flow into the fluid circuit when the refill tank valve (82) is opened,
**characterized in that** the refill tank valve (82) is coupled to a central control system (92) or can be controlled from outside the body, and that the central control system (92) is configured in such a way that, in the event of fluid loss in the fluid circuit, it automatically drives the refill tank valve (82) into the open position.

2. The cardiac assistance device as claimed in claim 1, **characterized in that** at least one pressure accumulator (60) is arranged on the supply side between pump (80) and chambers (24, 126, 226, 326), into which pressure accumulator (60) the pump (80) feeds pressure fluid, wherein the valves are arranged downstream from the pressure accumulator (60), and/or **in that** at least one pressure fluid collection chamber (204) is arranged on the discharge side between chambers (24, 126, 226, 326) and pump, and all of the lines leading from the chambers (24, 126, 226, 326) to the pump (80) open into this pressure fluid collection chamber (204).

3. The cardiac assistance device as claimed in claim 2, **characterized in that** a pressure sensor (94) is provided, which determines the pressure in the pressure accumulator (60).

4. The cardiac assistance device as claimed in claim 3, **characterized in that** the pressure sensor (94) is coupled to a control system (92) of the pump (80), which control system (92) is configured in such a way that the speed of rotation of the pump (80) is changed when the pressure in the pressure accumulator (60) drops below a predefined minimum pressure or exceeds a predefined maximum pressure.

5. The cardiac assistance device as claimed in one of the preceding claims, **characterized in that** a dedicated supply line and/or a dedicated discharge line issues from each chamber (24, 126, 226, 326).

6. The cardiac assistance device as claimed in claim 5, **characterized in that** on the discharge side the chambers (24, 126, 226, 326) have a direct fluidic connection to the pump (80), and/or **in that** the device is designed as an open system in which the pump (80) sucks in air from the atmosphere, and the air from the chambers (24, 126, 226, 326) is released into the atmosphere.

7. The cardiac assistance device as claimed in one of the preceding claims, **characterized in that** a control system (92) of the valves (54, 56, 58) and an emergency sensor system coupled thereto are provided, wherein the control system (92) is configured in such a way that, when leakage from a chamber (54, 56, 58) is detected, it permanently closes the valves (54, 56, 58) assigned to the defective chamber.

8. The cardiac assistance device as claimed in one of the preceding claims, **characterized in that**, in addition to the pump (80), an emergency pump (180) is provided along with a control system (92) of the pumps (80, 180), wherein the control system (92) is configured in such a way that it activates the emergency pump (180) when a defect of the running pump (80) is detected.

9. The cardiac assistance device as claimed in one of the preceding claims, **characterized in that** pressure sensors (102, 104, 106, 108) on the heart side are coupled to a control system (92) for valves (54, 56, 58) and/or the pump (80).

10. The cardiac assistance device as claimed in one of the preceding claims, wherein the refill tank (84) is preferably made of metal, in particular titanium, glass or plastic, or consists of a sandwich structure made of these materials.

11. The cardiac assistance device as claimed in one of the preceding claims, **characterized in that** the fluid circuit has a transcutaneously connectable refill interface (86), which is designed in particular as a passive valve, wherein the refill interface can be coupled releasably, preferably by a screw fastening or bayonet catch, to a refill tool that is connectable from the outside.

12. The cardiac assistance device as claimed in one of the preceding claims, **characterized in that** a central subcutaneous control system (92) is provided for the pump (80) and the valves (54, 56, 58), which control system (92) has a receiver (116) and can be reprogrammed via the receiver (116) and/or has a transmitter (116) for reading out data.

13. The cardiac assistance device as claimed in claim 12, **characterized by** an extracorporeal control unit (118) that can be coupled wirelessly to the subcutaneous control system (92) and has an Internet interface.

14. The cardiac assistance device as claimed in one of the preceding claims, **characterized in that** a central control system (92) for the pump (80) and the valves (54, 56, 58) is provided, which is coupled to pressure sensors (96 - 108) for determining the chamber pressure and/or the blood pressure, wherein the control system (92) is configured in such a way that, in the event of a constantly reduced chamber pressure and/or blood pressure, it drives the valves (54, 56, 58) such that the chambers (24, 126, 226, 326) are emptied less completely and/or hitherto unused chambers (24, 126, 226, 326) are filled.

## Revendications

1. Dispositif d'assistance cardiaque comprenant un composant de support entourant le coeur, sur lequel sont prévues du côté interne plusieurs chambres adjacentes (24 ; 126, 226, 326) pouvant être gonflées et dégonflées, par le biais desquelles une paroi du côté interne (28) peut être déplacée vers l'intérieur, les chambres (24 ; 126, 226, 326) étant en liaison fluidique par le biais de lignes avec au moins une pompe (80), plusieurs soupapes d'afflux du côté de l'afflux étant prévues entre la pompe (80) et les chambres (24 ; 126, 226, 326), au moins des chambres individuelles (24 ; 126, 226, 326) étant associées à une soupape d'afflux propre ou des groupes de chambres (24 ; 126, 226, 326) étant associés à des soupapes d'afflux propres, afin de commander les chambres (24 ; 126, 226, 326) individuellement ou par groupes indépendamment,
un réservoir de remplissage de fluide intracorporel (84) pouvant être accouplé au circuit de fluide par le biais d'une soupape de réservoir de remplissage intracorporelle (82), afin que du fluide puisse s'écouler dans le circuit de fluide lorsque la soupape de réservoir de remplissage (82) est ouverte,
**caractérisé en ce que** la soupape de réservoir de remplissage (82) est accouplée à une commande centrale (60) ou peut être commandée à l'extérieur du corps et la commande centrale (60) est réalisée de telle sorte qu'elle commande automatiquement la soupape de réservoir de remplissage (82) dans la position ouverte en cas de perte de fluide dans le circuit de fluide.

2. Dispositif d'assistance cardiaque selon la revendication 1, **caractérisé en ce qu'**entre la pompe (80) et les chambres (24 ; 126, 226, 326) est disposé, du côté de l'afflux, au moins un accumulateur de pression (60) dans lequel la pompe (80) injecte du fluide sous pression, les soupapes étant disposées en aval de l'accumulateur de pression (60), et/ou **en ce qu'**entre les chambres (24 ; 126, 226, 326) et la pompe est disposée, du côté aval, au moins une chambre de collecte de fluide sous pression (204), dans laquelle débouchent toutes les lignes conduisant depuis les chambres (24 ; 126, 226, 326) dans la direction de la pompe (80).

3. Dispositif d'assistance cardiaque selon la revendication 2, **caractérisé en ce qu'**un capteur de pression (94) déterminant la pression dans l'accumulateur de pression (60) est prévu.

4. Dispositif d'assistance cardiaque selon la revendication 3, **caractérisé en ce que** le capteur de pression (94) est accouplé à une commande (60) de la pompe (80) qui est réalisée de telle sorte que lors d'un dépassement par le dessous/par le haut d'un minimum de pression, respectivement d'un maximum de pression prédéfinis dans l'accumulateur de pression (60), la vitesse de rotation de la pompe (80) est modifiée.

5. Dispositif d'assistance cardiaque selon l'une quelconque des revendications précédentes, **caractérisé en ce qu'**une ligne d'afflux propre et/ou une ligne d'évacuation propre partent de chaque chambre (24 ; 126, 226, 326).

6. Dispositif d'assistance cardiaque selon la revendication 5, **caractérisé en ce que** du côté aval, les chambres (24 ; 126, 226, 326) sont connectées fluidiquement directement à la pompe (80) et/ou **en ce que** le dispositif est réalisé sous forme de système ouvert dans lequel la pompe (80) aspire de l'air de l'atmosphère et l'air est évacué hors des chambres (24 ; 126, 226, 326) dans l'atmosphère.

7. Dispositif d'assistance cardiaque selon l'une quelconque des revendications précédentes, **caractérisé en ce qu'**une commande (60) des soupapes (54, 56, 58) et un système de capteurs de secours accouplé à celle-ci sont prévus, la commande (60) étant réalisée de telle sorte que lors de la détection d'une fuite d'une chambre (24 ; 126, 226, 326), elle ferme de manière permanente les soupapes (54, 56, 58) associées à la chambre défectueuse.

8. Dispositif d'assistance cardiaque selon l'une quelconque des revendications précédentes, **caractérisé en ce qu'**en plus de la pompe (80), il est prévu une pompe de secours (180) ainsi qu'une commande (60) des pompes (80, 180), la commande (60) étant réalisée de telle sorte qu'elle active la pompe de secours (180) lors de la détection d'un défaut de la pompe courante (80).

9. Dispositif d'assistance cardiaque selon l'une quelconque des revendications précédentes, **caractérisé en ce que** des capteurs de pression du côté du coeur (102, 104, 106, 108) sont accouplés à une commande (60) pour des soupapes (54, 56, 58) et/ou pour la pompe (80).

10. Dispositif d'assistance cardiaque selon l'une quelconque des revendications précédentes, dans lequel le réservoir de remplissage (84) se compose de métal, notamment de titane, de verre ou de plastique, ou d'une construction en sandwich constituée de ces matériaux.

11. Dispositif d'assistance cardiaque selon l'une quelconque des revendications précédentes, **caractérisé en ce que** le circuit de fluide présente une interface de remplissage (86) pouvant être raccordée par voie transcutanée, qui est réalisée notamment sous forme de soupape passive, l'interface de remplissage pouvant de préférence être accouplée de manière amovible par le biais d'une fermeture vissée ou à baïonnette à un outil de remplissage pouvant être raccordé depuis l'extérieur.

12. Dispositif d'assistance cardiaque selon l'une quelconque des revendications précédentes, **caractérisé en ce qu'**une commande centrale sous-cutanée (60) pour la pompe (80) et les soupapes (54, 56, 58) est prévue, laquelle présente un récepteur (116) et est réalisée de manière reprogrammable par le biais du récepteur (116) et/ou laquelle présente un émetteur (116) pour lire des données.

13. Dispositif d'assistance cardiaque selon la revendication 12, **caractérisé par** une unité de commande extracorporelle (118) pouvant être accouplée sans câble à la commande sous-cutanée (60), laquelle présente une interface internet.

14. Dispositif d'assistance cardiaque selon l'une quelconque des revendications précédentes, **caractérisé en ce qu'**une commande centrale (60) pour la pompe (80) et les soupapes (54, 56, 58) est prévue, laquelle est accouplée à des capteurs de pression (96 - 108) pour déterminer la pression dans les chambres et/ou la pression sanguine, la commande (60) étant réalisée de telle sorte qu'elle commande les soupapes (54, 56, 58) dans le cas d'une réduction constante de la pression dans les chambres et/ou de la pression sanguine, de telle sorte que les chambres (24 ; 126, 226, 326) soient moins complètement vidées et/ou que des chambres (24 ; 126, 226, 326) jusqu'alors non utilisées soient remplies.
